(19) Europäisches Patentamt
European Patent Office
Office européen des brevets

(11) Veröffentlichungsnummer : **0 054 701**
**B1**

(12) # EUROPÄISCHE PATENTSCHRIFT

(45) Veröffentlichungstag der Patentschrift : **05.03.86**

(51) Int. Cl.[4] : **C 09 K 3/14**, A 61 K 7/00, A 61 K 7/16, C 09 G 1/02

(21) Anmeldenummer : **81109025.7**

(22) Anmeldetag : **27.10.81**

(54) **Abrasiv wirkende Mittel und deren Verwendung.**

(30) Priorität : **22.12.80 DE 3048369**

(43) Veröffentlichungstag der Anmeldung : **30.06.82 Patentblatt 82/26**

(45) Bekanntmachung des Hinweises auf die Patenterteilung : **05.03.86 Patentblatt 86/10**

(84) Benannte Vertragsstaaten : **AT BE CH DE FR GB IT LI LU NL SE**

(56) Entgegenhaltungen :
**DE-A- 2 758 414**
**DE-A- 2 758 415**
**DE-A- 2 925 969**

(73) Patentinhaber : **Fraunhofer-Gesellschaft zur Förderung der angewandten Forschung e.V.**
**Leonrodstrasse 54**
**D-8000 München 19 (DE)**

(72) Erfinder : **Schmidt, Helmut, Dr.**
**Tilman-Riemenschneider-Strasse 15**
**D-8706 Höchberg (DE)**
Erfinder : **Kaiser, Alfred, Dr.**
**Am Rosengarten 18**
**D-8702 Kist (DE)**

(74) Vertreter : **Barz, Peter, Dr. et al**
**Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8**
**D-8000 München 40 (DE)**

**EP 0 054 701 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft abrasiv wirkende Mittel, die sich sowohl für medizinische oder kosmetische Zwecke, z. B. zur Aknebehandlung, als auch für technische Zwecke, z. B. als Poliermittel für empfindliche Oberflächen, eignen.

Abrasiv wirkende Mittel enthalten gewöhnlich einen schleifenden oder scheuernden Bestandteil sowie in Anpassung an den jeweiligen Verwendungszweck unterschiedliche Trägerstoffe, Hilfsstoffe und Aktivkomponenten. In vielen Anwendungsbereichen wird gefordert, daß die Schleif- oder Scheuerwirkung der abrasiv wirkenden Mittel variabel eingestellt werden kann ; vor allem besteht in manchen Anwendungsbereichen Bedarf für Mittel, deren Schleif- oder Scheuerwirkung während der Anwendung in definierter Weise nachläßt.

Dieses Problem besteht z. B. bei Hautpflegemitteln zur Aknebehandlung. Wollte man in derartigen Mitteln übliche Schleifmittel verwenden, z. B. Sand, Silicate, Kieselgele oder Aluminiumoxid, so würden aufgrund der anhaltenden Scheuerwirkung vor allem bei übermäßiger Anwendung Hautschäden auftreten. Herkömmliche Präparate enthalten deshalb z. B. in einer üblichen Seifengrundlage Natriumtetraborat (Borax) als Abrasivum. Natriumtetraborat hat den Vorteil, daß es eine für den Anwendungszweck brauchbare Härte aufweist und seine Scheuerwirkung aufgrund des Abriebs in geeigneter Weise abnimmt, so daß Hautschädigungen vermieden werden. Allerdings hat sich in jüngerer Zeit herausgestellt, daß Borverbindungen gesundheitlich nicht unbedenklich sind ; so wurden z. B. die Borsalben im wesentlichen vollständig vom Markt genommen.

Es ist auch bereits bekannt, in Hautpflegemitteln der genannten Art Natriumchlorid als Abrasivum zu verwenden. In diesem Fall werden jedoch Hautreizungen verursacht und besonders an schadhaften Stellen der Haut tritt schmerzhaftes Brennen auf.

Überraschenderweise wurde nun gefunden, daß poröse, organisch modifizierte Kieselsäureheteropolykondensate ausgezeichnete Abrasiva sind, deren Schleif- bzw. Scheuerwirkung in definierter Weise sowohl absolut als auch als Funktion der Zeit einstellbar ist.

Aufgrund dieser Eigenschaften eignen sich die Schleifmittel der Erfindung nicht nur für medizinische oder kosmetische Präparate, z. B. Aknemittel oder Zahnpasten, sondern auch für technische Anwendungen, z. B. in Lackpflegemitteln oder Poliermitteln für empfindliche Oberflächen.

Die als Schleifmittel eingesetzten porösen Kieselsäureheteropolykondensate werden hergestellt durch hydrolytische Polykondensation von einem oder mehreren Silanen oder Polysiloxanen, wobei mindestens ein Silan oder Polysiloxan einen oder mehrere an Silicium gebundene Organoreste aufweist.

Bevorzugte Kieselsäureheteropolykondensate sind Copolykondensate von vollständig hydrolysierbaren Silanen, z. B. Tetraalkoxysilanen oder Polyalkoxysiloxanen, mit Organosilanen. Hierbei wird der Anteil der vollständig hydrolysierbaren Silane auf einen ausreichend hohen Wert eingestellt, damit ein poröses Produkt entsteht.

In einer besonders bevorzugten Ausführungsform verwendet man als Schleifmittel Kieselsäureheteropolykondensate, die hergestellt worden sind durch Hydrolyse und Polykondensation von

a) mindestens einem siliciumfunktionellen Silan der allgemeinen Formel I

$$SiX_4 \qquad (I)$$

in der X Wasserstoff, Halogen, Alkoxy, Acyloxy oder $-NR_2$ (R = Wasserstoff und/oder Alkyl) bedeutet, jedoch nicht alle Reste X Wasserstoff sind,

b) mindestens einem Organosilan der allgemeinen Formel II

$$SiR'_aX_{(4-a)} \qquad (II)$$

in der X die vorstehende Bedeutung hat, R' Alkyl, Alkenyl, Aryl, Aralkyl oder Alkylaryl bedeutet und a den Wert 1, 2 oder 3 hat,

c) gegebenenfalls mindestens einem organofunktionellen Silan der allgemeinen Formel III

$$SiR'_bX_c(R''Y)_{(4-b-c)} \qquad (III)$$

in der R' und X die vorstehende Bedeutung haben, R″ geradkettiges oder verzweiges Alkylen, das durch Sauerstoff- oder Schwefelatome oder —NH-Gruppen unterbrochen sein kann, Phenylen, Alkylphenylen oder Alkylenphenylen darstellt, Y Halogen oder eine gegebenenfalls substituierte Amino-, gegebenenfalls substituierte Anilino-, Aldehyd-, Keto-, Carboxy-, Hydroxy-, Mercapto-, Cyano-, Hydroxyphenyl-, Diazo-, Carbonsäurealkylester-, Sulfonsäure- ($SO_3H$) oder Phosphorsäuregruppe ($PO_3H_2$) bedeutet, b den Wert 0, 1 oder 2, c den Wert 1, 2 oder 3 und (b + c) den Wert 1, 2 oder 3 haben, und/oder

d) gegebenenfalls mindestens einem im Reaktionsmedium löslichen schwer-flüchtigen Oxid oder mindestens einer im Reaktionsmedium löslichen, ein schwer-flüchtiges Oxid bildenden Verbindung eines Elementes der Hauptgruppen Ia bis Va oder der Nebengruppen IVb oder Vb des Periodensystems (CRC Handbook of Chemistry and Physics, CRC Press Inc.) in Gegenwart mindestens der zur Hydrolyse

stöchiometrisch erforderlichen Wassermenge und gegebenenfalls in Gegenwart eines Kondensationskatalysators und/oder eines organischen Lösungsmittels, eventuelles Abtrennen des organischen Lösungsmittels, Trocknen und Zerkleinern des erhaltenen Kieselsäureheteropolykondensats.

Das auf diese Weise hergestellte Kieselsäureheteropolykondensat enthält vorzugsweise, bezogen auf Oxideinheiten, 35 bis 90 Gewichtsprozent der Komponente (a), 10 bis 50 Gewichtsprozent der Komponente (b), 0 bis 15 Gewichtsprozent der Komponente (c) und 0 bis 40 Gewichtsprozent der Komponente (d).

Diese und ähnliche Kieselsäureheteropolykondensate sowie Verfahren zu ihrer Herstellung sind z. B. in den DE-OS 27 58 415 und 29 25 969 beschrieben. Bezüglich bevorzugter Produktzusammensetzungen und spezieller Beispiele für die Komponenten (a) bis (d) wird auf diese Patentveröffentlichungen verwiesen.

Bei der Auswahl der Komponenten (a) bis (d) und insbesondere bei der Wahl der Oxidkomponente (d) versteht es sich, daß für den medizinischen oder kosmetischen Anwendungsbereich nur nicht-toxische und kosmetisch verträgliche Substanzen verwendet werden. Bei der Oxidkomponente (d) sind dies insbesondere Oxide und oxidbildende Verbindungen von Al, Ca, Mg, Ti, Zr, Zn und Si.

Die auf die vorstehend beschriebene Weise erhaltenen Kieselsäureheteropolykondensate können nach der Auskondensation gewaschen, getrocknet, zerkleinert und fraktioniert werden.

Die erfindungsgemäßen Schleifmittel haben üblicherweise eine Korngröße von z. B. 0,01 bis 1 mm, vorzugsweise 0,1 bis 0,5 mm und insbesondere 0,2 bis 0,4 mm. Für spezielle Anwendungszwecke können auch Korngrößen außerhalb dieser Grenzwerte angewandt werden.

Die Härte der Schleifmittel variiert zwischen 1 und 5 auf der Mohs-Skala. Die Härte kann z. B. eingestellt werden über die Zusammensetzung der Kieselsäureheteropolykondensate und die Kondensationsgeschwindigkeit. So ergibt ein hoher Anteil an den Komponenten (a) und/oder (d) härtere Produkte ; gleichermaßen führt eine niedrige Kondensationsgeschwindigkeit zu kompakten harten Produkten mit grober Körnung, während eine hohe Kondensationsgeschwindigkeit voluminöse weiche Pulver mit feiner Körnung ergibt.

Die abrasiv wirkenden Mittel der Erfindung enthalten neben dem Schleifmittel übliche Trägerstoffe, Hilfsstoffe und/oder Aktivkomponenten. Der Schleifmittelgehalt kann zwischen 1 bis 99 Gewichtsprozent variieren, beträgt jedoch üblicherweise 2 bis 50, vorzugsweise 3 bis 30 und insbesondere 5 bis 15 Gewichtsprozent, bezogen auf das Gewicht des Gesamtmittels.

Die Mittel können z. B. als wässrige oder ölige Suspensionen, Schüttelmixturen, Emulsionssalben, wasserfreie Salben, Pasten, Cremes, Gele, Pulver, Puder oder Schaumpräparate vorliegen. Ferner können sie auf Papiere oder Vliesstoffe aus Cellulose-, Textil-, Kunstharz- oder Glasfasern aufgebracht bzw. darin eingebettet werden. Die Schleifmittel können aber auch mit einem Gasstrom gegen das zu behandelnde Substrat gestrahlt werden.

Die für diese und andere Anwendungsformen verwendeten Träger können fest, halbfest, flüssig oder gasförmig sein. Spezielle Beispiele sind Wasser, wässrige Lösungen, organische Lösungsmittel, z. B. Alkohole, Glykole, Polyglykoläther, Ketone, aliphatische und aromatische Kohlenwasserstoffe oder Halogenkohlenwasserstoffe, Erdölfraktionen, Paraffinöl und Öle oder Fette tierischen oder pflanzlichen Ursprungs ; Tone, Kaolin, Bentonit, Talkum, Diatomeenerde, Titandioxid, Calciumcarbonat oder Stärke ; Luft, Inertgase, Kohlendioxid oder Freone.

Für medizinische oder kosmetische Zwecke werden Salbengrundlagen bevorzugt, z. B. W/O- oder O/W-Emulsionen, Paraffinkohlenwasserstoffe, wie Vaseline oder Paraffin, Silicon- oder Fettgrundlagen. Ebenfalls geeignet sind waschaktive Grundlagen, z. B. nichtionische, kationische, amphotere oder vorzugsweise anionische Tenside, herkömmliche Seifen oder Syndets. Spezielle verwendbare Tenside sind die Handelsprodukte TEXAPON$^R$ von Henkel, HOSTAPON$^R$ von Hoechst, LAMEPON$^R$ von der Chemischen Fabrik Grünau GmbH und LATHANOL$^R$ von der Allied Chemical Corporation.

Die erfindungsgemäßen Mittel können je nach dem Verwendungszweck die verschiedensten Hilfsstoffe enthalten, z. B. Puffer, Bindemittel, wie Cellulosederivate, Stärke, Gelatine, Gummiarabicum oder Traganth, anionische, kationische, neutrale oder amphotere Polymerisate, Verdickungsmittel, z. B. Cellulosederivate, Füllstoffe oder Streckmittel, z. B. Kieselgele, Silicate, Carbonate, Phosphate oder Kohlenhydrate, Hautweichmacher, Emulgatoren, z. B. die vorstehend genannten Tenside, Alkaliphosphate oder Polyphosphate, Färbemittel, z. B. organische Farbstoffe oder organische oder anorganische Pigmente, Siliconöle, Bleichmittel, Gleitmittel, z. B. Metallseifen, Duftstoffe, Desodorants, Geschmackskorrigentien, herkömmliche Schleifmittel und/oder natürliche oder synthetische Wachse, wie Bienenwachs, Carnauba-wachs oder Montanwachs.

Gegebenenfalls können den Mitteln auch Aktivkomponenten zugesetzt werden, z. B. pharmazeutische Wirkstoffe, Desinfektionsmittel, Konservierungsmittel, insektizide, bakterizide oder fungizide Mittel, Antioxidantien oder korrosionshemmende Zusätze.

Die abrasiv wirkenden Mittel der Erfindung werden in den jeweiligen Anwendungsgebieten auf herkömmliche Weise eingesetzt ; so z. B. im medizinischen oder kosmetischen Bereich als Aknemittel oder Zahnpasten, im technischen Bereich als Putz-, Reinigungs- oder Scheuermittel, als Lackreiniger oder Polituren.

Im folgenden wird die Erfindung anhand von Aknemitteln näher erläutert. Diese Mittel enthalten das Schleifmittel in einer Grundlage aus waschaktiven Substanzen. Darüberhinaus können z. B. reduzierend

wirkende Stoffe, wie Schwefel oder Resorcin, adstringierende Mittel, wie Zinkoxid, desinfizierende Mittel, z. B. Antibiotika oder Hexachlorophen, entzündungshemmende Mittel, z. B. Corticosteroide, abschwellende oder juckreizstillende Mittel, z. B. sulfurierte Schieferöle, Teere, Campher oder Phenol, oder keratolytisch wirkende Substanzen, z. B. Salicylsäure, zugesetzt werden.

Beispiel 1

95,6 ml Tetramethoxysilan Si $(OCH_3)_4$ und 26,2 ml Dimethyldiethoxysilan $(CH_3)_2Si(OC_2H_5)_2$ werden in 122 ml Methanol gelöst und unter Rühren bei Raumtemperatur mit 52 ml 1 N $NH_3$ versetzt. Nach dem Verfestigen der Reaktionsmischung wird mit heißem destilliertem Wasser gewaschen, bis das Waschwasser nicht mehr alkalisch reagiert. Das Kondensat wird bei 110 °C getrocknet und anschließend zerkleinert und fraktioniert.

Die Kornfraktion 0,2-0,4 mm wird in einer Menge von 15 Gewichtsprozent in LAMEPON S^R (Eiweiß-Fettsäurekondensat ; wäßrige Lösung mit einem Aktivgehalt von 32 % ; Hersteller : Chem. Fabr. Grünau GmbH) als Salbengrundlage eingearbeitet.

Beispiel 2

86,1 ml Tetramethoxysilan $Si(OCH_3)_4$ und 35,7 ml Dimethyldiethoxysilan $(CH_3)_2Si(OC_2H_5)_2$ werden in 200 ml Methanol gelöst und mit 100 ml 12 N HCl versetzt. Nach kurzem Rühren wird bei Raumtemperatur stehengelassen. Das erstarrte Produkt wird so oft mit destilliertem Wasser aufgekocht und abfiltriert, bis das Filtrat pH-neutral ist. Nach dem Trocknen bei 110 °C wird das Produkt zerkleinert und fraktioniert.

Die Kornfraktion 0,1-0,3 mm wird in einer Menge von 10 Gewichtsprozent in LATHANOL^R (Natriumlaurylsulfoacetat ; Hersteller : Allied Chem. Corp.) als Salbengrundlage eingearbeitet.

Beispiel 3

Eine Syndet-Seifenpaste wird aus folgenden Bestandteilen hergestellt :

| | Gew.- % |
|---|---|
| Kieselsäureheteropolykondensat | 15 |
| IRGASAN DP 300^R (Bakterizid von Ciba Geigy) | 0,1 |
| ARLACEL 165^R (Emulgator von Atlas) | 4 |
| TEXAPON N 25^R (Tensid von Henkel) | 6 |
| LAMEPON S^R (Eiweiß-Fettsäurekondensat der Chemischen Fabrik Grünau GmbH) | 6 |
| LATHANOL^R-Pulver (Natriumlaurylsulfoacetat der Allied Chemical Corp.) | 6 |
| HOSTAPON CT^R-Teig (anionisches Tensid von Hoechst) | 40 |
| AEROSIL (Siliciumdioxid von Degussa) | 1 |
| Parfums, Farbstoffe | ad libitum |
| Aqua dest. | ad 100 |

Beispiel 4

Eine Seife wird aus 15 Gew.- % Kieselsäureheteropolykondensat und einem herkömmlichen Seifenkörper ad 100 % hergestellt.

**Patentansprüche**

1. Abrasiv wirkende Mittel, enthaltend ein poröses, organisch modifiziertes Kieselsäureheteropolykondensat als Schleifmittel neben üblichen Trägerstoffen und/oder Aktivkomponenten und/oder Hilfsstoffen zur Verwendung für medizinische und kosmetische Zwecke.

2. Mittel nach Anspruch 1, dadurch gekennzeichnet, daß das Schleifmittel eine Korngröße von 0,01 bis 1 mm, vorzugsweise 0,1 bis 0,5 mm und insbesondere 0,2 bis 0,4 mm hat.

3. Mittel nach Anspruch 1 oder 2, dadurch gekennzeichnet, daß das Schleifmittel eine Härte von 1 bis 5 auf der Mohs-Skala hat.

4. Mittel nach einem der Ansprüche 1 bis 3, dadurch gekennzeichnet, daß das Schleifmittel dadurch hergestellt worden ist, daß man

a) mindestens ein siliciumfunktionelles Silan der allgemeinen Formel I

$$SiX_4 \qquad (I)$$

in der X Wasserstoff, Halogen, Alkoxy, Acyloxy oder $—NR_2$ (R = Wasserstoff und/oder Alkyl) bedeutet, jedoch nicht alle Reste X Wasserstoff sind,

b) mindestens ein Organosilan der allgemeinen Formel II

$$SiR'_aX_{(4-a)} \quad (II)$$

in der X die vorstehende Bedeutung hat, R' Alkyl, Alkenyl, Aryl, Aralkyl oder Alkylaryl bedeutet und a den Wert 1, 2 oder 3 hat,

c) gegebenenfalls mindestens ein organofunktionelles Silan der allgemeinen Formel III

$$SiR'_bX_c(R''Y)_{(4-b-c)} \quad (III)$$

in der R' und X die vorstehende Bedeutung haben, R'' geradkettiges oder verzweigtes Alkylen, das durch Sauerstoff- oder Schwefelatome oder —NH-Gruppen unterbrochen sein kann, Phenylen, Alkylphenylen oder Alkylenphenylen darstellt, Y Halogen oder eine gegebenenfalls substituierte Amino-, gegebenenfalls substituierte Anilino-Aldehyd-, Keto-, Carboxy-, Hydroxy-, Mercapto-, Cyano-, Hydroxyphenyl-, Diazo-, Carbonsäurealkylester-, Sulfonsäure- ($SO_3H$) oder Phosphorsäuregruppe ($PO_3H_2$) bedeutet, b den Wert 0, 1 oder 2, c den Wert 1, 2 oder 3 und (b + c) den Wert 1, 2 oder 3 haben, und/oder

d) gegebenenfalls mindestens ein im Reaktionsmedium lösliches schwerflüchtiges Oxid oder mindestens eine im Reaktionsmedium lösliche, ein schwerflüchtiges Oxid bildende Verbindung eines Elementes der Hauptgruppen Ia bis Va oder der Nebengruppen IVb oder Vb des Periodensystems in Gegenwart mindestens der zur Hydrolyse stöchiometrisch erforderlichen Wassermenge und gegebenfalls in Gegenwart eines Kondensationskatalysators und/oder eines organischen Lösungsmittels hydrolysiert und polykondensiert, das erhaltene Kieselsäureheteropolykondensat nach eventuellem Abtrennen des organischen Lösungsmittels trocknet und zerkleinert.

5. Mittel nach Anspruch 4, dadurch gekennzeichnet, daß das erhaltene Kieselsäureheteropolykondensat, bezogen auf Oxideinheiten, 35 bis 90 Gewichtsprozent der Komponente (a), 10 bis 50 Gewichtsprozent der Komponente (b), 0 bis 15 Gewichtsprozent der Komponente (c) und 0 bis 40 Gewichtsprozent der Komponente (d) enthält.

6. Verwendung der Mittel nach einem der Ansprüche 1 bis 5 zur Herstellung eines pharmazeutischen Präparates zur Aknebehandlung.

7. Verwendung von abrasiv wirkenden Mitteln, die ein poröses, organisch modifiziertes Kieselsäureheteropolykondensat als Schleifmittel neben üblichen Trägerstoffen und/oder Aktivkomponenten und/oder Hilfsstoffen enthalten, als Schleif-, Scheuer-, Lackpflege- oder Poliermittel.

## Claims

1. An abrasive composition comprising a porous, organo-modified silicic acid heteropolycondensate as abrasive in addition to conventional carriers and/or active components and/or adjuvants

for use for medical and cosmetic purposes.

2. The composition of claim 1, wherein the abrasive has a particle size of from 0.01 to 1 mm, preferably 0.1 to 0.5 mm, and more preferably, 0.2 to 0.4 mm.

3. The composition of claim 1 or 2, wherein the abrasive has a hardness of 1 to 5 on the Mohs scale.

4. The composition of any one of claims 1 to 3, wherein the abrasive has been prepared by hydrolytic polycondensation of :

a) at least one silicon-functional silane of the formula (I) :

$$SiX_4 \quad (I)$$

wherein X is hydrogen, halogen, alkoxy, acyloxy, or an —$NR_2$ group, in which R is hydrogen and/or alkyl, with the proviso that not all of X are hydrogen ;

b) at least one organo-silane of the formula (II) :

$$SiR'_aX_{(4-a)} \quad (II)$$

wherein X is as defined above ; R' is alkyl, alkenyl, aryl, aralkyl or alkylaryl ; and a is an integer of 1, 2 or 3 ;

c) optionally at least one organo-functional silane of the formula (III) :

$$SiR'_bX_c(R''Y)_{(4-b-c)} \quad (III)$$

wherein R' and X are as defined above ; R'' is straight-chain or branched alkylene, optionally interrupted by oxygen or sulfur atoms or —NH-groups ; phenylene, alkylphenylene or alkylenephenylene ; Y is halogen, unsubstituted or substituted amino, unsubstituted or substituted anilino, or an aldehyde ; keto, carboxy, hydroxy, mercapto, cyano, hydroxyphenyl, diazo, carboxylic acid alkyl ester, sulfonic acid ($SO_3H$) or phosphoric acid ($PO_3H_2$) group ; b is 0 or 1 or 2 ; c is 1 or 2 or 3 ; and (b + c) is 1 or 2 or 3 ; and/or

d) optionally at least one substantially involatile oxide of an element of the main groups Ia to Va or the secondary groups IVb or Vb of the periodic table, which is soluble in the reaction medium, or at least one compound which is soluble in the reaction medium and is capable of forming such a substantially involatile oxide,

in the presence of at least the amount of water stoichio-metrically required for hydrolysis and, optionally, in the presence of a condensation catalyst and/or an organic solvent, followed by removing any solvent present and drying and comminuting the silicic acid heteropolycondensate obtained.

5. The composition of claim 4, wherein the silicic acid heteropolycondensate obtained comprises from 35 to 90 % by weight of component (a), from 10 to 50 % by weight of component (b), from 0 to 15 % by weight of component (c), and from 0 to 40 % by weight of component (d), based on the oxide units.

6. The use of a composition according to any one of claims 1 to 5 for preparing a pharmaceutical preparation useful for the treatment of acne.

7. The use of abrasive compositions comprising a porous, organo-modified silicic acid heteropolycondensate as abrasive in addition to conventional carriers and/or active components and/or adjuvants as scouring agents, cleansers for enamel or polishing agents.

**Revendications**

1. Produit à action abrasive, contenant un produit poreux d'hétéropolycondensation de l'acide silicique, à modification organique, comme agent de polissage, en plus des supports usuels et/ou de constituants actifs usuels et/ou adjuvants, destiné à servir à des fins médicinales et cosmétiques.

2. Produit selon la revendication 1, caractérisé en ce que l'agent de polissage présente une grosseur de grains de 0,01 à 1 mm, avantageusement 0,1 à 0,5 mm et en particulier 0,2 à 0,4 mm.

3. Produit selon la revendication 1 ou 2, caractérisé en ce que l'agent de polissage possède une dureté de 1 à 5 sur l'échelle de Mohs.

4. Produit selon l'une des revendications 1 à 3, caractérisé en ce que l'agent de polissage a été produit par hydrolyse et polycondensation de :

a) au moins un silane comportant les groupes fonctionnels fixés sur le silicium et répondant à la formule générale I :

$$SiX_4 \qquad (I)$$

(dans laquelle X représente un atome d'hydrogène ou d'halogène ou un groupe alcoxy, acyloxy, ou $—NR_2$ (R représentant un atome d'hydrogène et/ou un groupe alkyle), l'ensemble des restes X ne représentant pas simultanément chacun un atome d'hydrogène),

b) au moins un organosilane de formule générale II :

$$SiR'_aX_{(4-a)} \qquad (II)$$

dans laquelle X a le sens ci-dessus, R' représente un groupe alkyle, alcényle, aryle, aralkyle ou alkylaryle et a vaut 1, 2 ou 3,

c) éventuellement au moins un silane comportant des groupes organiques fonctionnels de formule générale III :

$$SiR'_bX_c(R''Y)_{(4-b-c)} \qquad (III)$$

dans laquelle R' et X ont le sens précité, R'' représente un groupe alkylène linéaire ou ramifié pouvant être interrompu par des atomes d'oxygène ou de soufre ou par des groupes —NH—, phénylène, alkylphénylène ou alkylène phénylène ; Y représente un halogène ou un groupe amino éventuellement substitué, un groupe anilino, aldéhyde, cétone, carboxy, hydroxy, mercapto, cyano, hydroxyphényle, diazo, ester alkylique d'acide carboxylique, acide sulfonique ($SO_3H$) ou acide phosphorique ($PO_3H_2$) éventuellement substitués ; b vaut 0, 1 ou 2 ; c vaut 1, 2 ou 3 et la somme (b + c) vaut 1, 2 ou 3, et/ou

d) éventuellement au moins un oxyde peu volatil, soluble dans le milieu de réaction ou au moins un composé générateur d'un oxyde peu volatil, soluble dans le milieu de réaction d'un élément des groupes principaux Ia à Va ou des sous-groupes IVb ou Vb du système périodique, en présence d'au moins la quantité d'eau stoechiométriquement nécessaire pour l'hydrolyse et éventuellement en présence d'un catalyseur de condensation et/ou d'un solvant organique, puis séchage après séparation éventuelle du solvant organique, du produit d'hétéropolycondensation d'acide silicique obtenu et fragmentation de ce produit.

5. Produit selon la revendication 4, caractérisé en ce que le produit d'hétéropolycondensation d'acide silicique obtenu contient, par rapport aux motifs oxydes, 35 à 90 % en poids du constituant (a), 10 à 50 % en poids du constituant (b), 0 à 15 % en poids du constituant (c) et 0 à 40 % en poids du constituant (d).

6. Utilisation du produit selon l'une des revendications 1 à 5 pour la fabrication d'une préparation pharmaceutique destinée au traitement de l'acné.

7. Utilisation des produits à action abrasive, qui contiennent un produit poreux d'hétéropolycondensation d'acide silicique à modification organique, comme agents de polissage, en plus des supports et/ou constituants actifs usuels, et/ou des adjuvants usuels, à titre de produits de polissage, de lustrage, d'entretien des vernis ou pour rendre poli et brillant.